# EUROPÄISCHE PATENTSCHRIFT

(11) **EP 1 232 393 B1**
(45) Veröffentlichungstag und Bekanntmachung des Hinweises auf die Patenterteilung: **11.06.2003**
(21) Anmeldenummer: 01925430.9
(22) Anmeldetag: 13.03.2001
(51) Int. Cl.: G01N 33/497, G01N 33/98

(54) **ATEMALKOHOLMESSGERÄT**
BREATH-ALCOHOL MEASURING INSTRUMENT
ETHYLOMETRE

(30) Priorität: 19.06.2000 DE 10030053
(43) Veröffentlichungstag der Anmeldung: 21.08.2002
(73) Patentinhaber: EnviteC-Wismar GmbH, 23966 Wismar (DE)
(72) Erfinder: LINDNER, Bernd, 23626 Ratekau (DE)
(74) Vertreter: Best, Michael, Dr.
(86) Internationale Anmeldenummer: EP0102787
(87) Internationale Veröffentlichungsnummer: WO01098778

(56) Entgegenhaltungen:
- EP-A- 0 752 584
- DE-A- 2 928 433
- US-A- 3 830 630

## Beschreibung

Die Erfindung betrifft ein neues Atemalkoholmeßgerät, das neben einer üblichen Vorrichtung zur Bestimmung des Alkoholgehalts in der Ausatemluft einen Sauerstoffsensor enthält, über den der Partialdruck des Kohlendioxidgehalts in der Ausatemluft bestimmt werden kann. Die Erfindung betrifft ebenfalls ein Verfahren, mit dem die Zuverlässigkeit der über die Alkoholkonzentration in der Ausatemluft bestimmten Blutalkoholkonzentrationswerte überprüft werden kann.

Atemalkoholmeßgeräte werden in der Praxis insbesondere bei Verkehrskontrollen häufig eingesetzt. Bei diesen Geräten wird der Alkoholgehalt in der Ausatemluft bestimmt und aus dem Alkoholgehalt in der Ausatemluft auf die Blutalkoholkonzentration geschlossen. Hierbei macht man sich zunutze, daß die Blutalkoholkonzentration in einem konstanten Gleichgewicht zur Konzentration des Alkohols in tiefer Lungenluft (Alveolarluft) ist. Die über ein Atemalkoholmeßgerät bestimmte Blutalkoholkonzentration entspricht aber nur dann in etwa der tatsächlich vorliegenden Blutalkoholkonzentration, wenn die untersuchte Person "normal" atmet. Durch eine geeignete Atemtechnik kann das Meßergebnis eines Atemalkoholmeßgeräts verfälscht werden. Es gibt dann nicht mehr die tatsächliche Blutalkoholkonzentration wieder.

Beispielsweise ist eine Verfälschung des Ergebnisses dann möglich, wenn die zu untersuchende Person während der Untersuchung flach atmet und nicht das gesamte Lungenvolumen in das Atemalkoholmeßgerät strömt. Während eine derartige Methode, das Meßergebnis zu verfälschen, dem Bedienpersonal unter Umständen noch auffallen kann, kann das Meßergebnis z.B. auch dadurch verfälscht werden, daß die zu untersuchende Person vor der Verwendung des Atemalkoholmeßgeräts hyperventiliert. Hierdurch ist der Alkoholgehalt in der Alveolarluft geringer als der Gleichgewichtswert, und das Atemalkoholmeßgerät zeigt einen zu niedrigen Wert an, obwohl während der Messung kein unnatürliches Atmen der zu untersuchenden Person beobachtet werden kann.

Umgekehrt kommt es bei Personen, die hypoventilieren, zu einer Anreicherung des Alkohols in der Alveolarluft und das Atemalkoholmeßgerät zeigt einen überhöhten Alkoholwert an. Schließlich gibt es Personen mit krankheitsbedingten Atemproblemen, beispielsweise Asthmatiker, bei denen ebenfalls das Meßergebnis des Atemalkoholmeßgeräts nicht notwendigerweise die korrekte Blutalkoholkonzentration wiederspiegelt.

Es gibt eine Reihe von Vorschlägen, Atemalkoholmeßgeräte bzw. Verfahren zu ihrer Verwendung so abzuändern, daß die vorstehenden Fehlerquellen minimiert werden. Insbesondere gibt es eine Reihe von Vorschlägen, wie abgeschätzt werden kann, ob der mit einem Atemalkoholmeßgerät ermittelte Blutalkoholkonzentrationswert dem realen Blutalkoholkonzentrationswert entspricht oder ob die Gefahr besteht, daß Abweichungen auftreten.

Hier kann beispielsweise auf die DE-A 29 28 433 verwiesen werden, die eine Einrichtung zum Steuern eines Atemalkoholmeßgeräts offenbart, bei der eine Fühlerstufe auf Druckschwankungen der Ausatemluft anspricht und ein der Amplitude dieser Druckschwankungen entsprechendes Signal erzeugt. Anstelle von Druckschwankungen in der Ausatemluft können auch Konzentrationsschwankungen einer Gaskomponente in der Ausatemluft bestimmt werden. Die Fühlerstufe ist dann beispielsweise ein CO₂-Fühler oder ein O₂-Fühler. Über die Druckschwankungen bzw. die Schwankungen im CO₂-Gehalt oder im O₂-Gehalt in der Ausatemluft eines Patienten wird sichergestellt, daß nur die Alveolarluft zur Alkoholmessung verwendet wird. Die Korrektur von Atemalkoholmeßgeräten über Druckschwankungen bzw. Schwankungen einer Gaskomponente in der Ausatemluft während eines Atemzugs haben sich in der Praxis jedoch als nicht ausreichend zuverlässig erwiesen. Auch ist die in der DE-A 29 28 433 beschriebene Einrichtung komplex und kostspielig.

Die EP-A 752 584 versucht nicht wie die DE-A 29 28 433 über Schwankungen in der Ausatemluft ein Atemalkoholmeßgerät so zu steuern, daß nur die Alveolarluft zur Bestimmung des Alkoholgehalts in der Ausatemluft verwendet wird. Diese Druckschrift offenbart ein Verfahren, mit dem festgestellt werden kann, ob der von einem Atemalkoholmeßgerät angegebene Wert verläßlich die Blutalkoholkonzentration wiedergibt, oder ob während der Messung beabsichtigt oder unbeabsichtigt Manipulationen auftraten. In dem Verfahren der EP-A 752 584 wird der Kohlendioxidgehalt in der Ausatemluft bestimmt und mit dem gleichzeitig gemessenen Alkoholgehalt in Bezug gesetzt. Sofern der Kohlendioxidgehalt unter einen bestimmten, vorher festgelegten Wert fällt, wird die Alkoholmessung als nicht zuverlässig angesehen.

Auch die US-A 3,830,630 offenbart ein Verfahren und eine Vorrichtung, bei der zunächst der Kohlendioxidgehalt der Ausatemluft bestimmt wird und nur wenn dieser Kohlendioxidgehalt den Grenzwert von 4,5% übersteigt, wird der Alkoholgehalt in der Ausatemluft bestimmt und für zuverlässig befunden.

Die in der EP-A 752 584 und der US-A 3,830,630 beschriebenen Verfahren weisen allerdings den Nachteil auf, daß zur genauen Bestimmung des Kohlendioxidgehalts in der Ausatemluft ein kostspieliger Kohlendioxidsensor benötigt wird. Die dort beschriebenen Verfahren können daher zwar auf recht einfache Art und Weise die Zuverlässigkeit eines mit einem Atemalkoholmeßgerät bestimmten Blutalkoholwerts vorhersagen, für den praktischen Einsatz, insbesondere bei Verkehrskontrollen, sind Vorrichtungen zur Durchführung dieses Verfahrens jedoch zu kostspielig, wenn eine genaue und schnelle Messung des Kohlendioxidgehalts gewünscht wird.

Es besteht daher weiterhin ein Bedarf nach einem einfach und kostengünstig durchzuführenden Verfahren zur Bestimmung der Zuverlässigkeit der mit einem Atemalkoholmeßgerät bestimmten Blutalkoholkonzentration, das sich insbesondere auch für den Masseneinsatz bei Verkehrskontrollen eignet, sowie nach preiswerten und einfachen Vorrichtungen zur Durchführung eines derartigen Verfahrens.

Es ist Aufgabe der vorliegenden Erfindung ein derartiges Verfahren bzw. eine derartige Vorrichtung zur Verfügung zu stellen. Das Verfahren und die Vorrichtung sollen insbesondere die Nachteile der entsprechenden Verfahren und Vorrichtungen des Standes der Technik nicht zeigen.

Diese Aufgabe wird durch den Gegenstand der Patentansprüche gelöst.

In der nicht vorveröffentlichten, nach dem PCT-Vertrag hinterlegten Anmeldung mit der Anmeldenummer PCT/EP00/03689 ist ein Verfahren zur Bestimmung des CO₂-Gehalts in der Atemluft sowie eine Beatmungsvorrichtung offenbart, die so ausgestaltet ist, daß das Verfahren damit durchgeführt werden kann. Das Verfahren beruht darauf, daß man mit einem schnellen Sauerstoffsensor den Sauerstoffpartialdruck beim Atmen bestimmt. Aus dem Sauerstoffpartialdruck kann dann auf den Kohlendioxidgehalt in der Atemluft geschlossen werden. Beträgt beispielsweise der Sauerstoffpartialdruck in der Einatemluft 21 kPa und fällt der Sauerstoffpartialdruck in der Ausatemluft bis auf 16 kPa, entspricht in erster Näherung die Differenz im Sauerstoffpartialdruck von 5 kPa dem Maximalwert des Kohlendioxidpartialdrucks in der Ausatemluft. Wichtige Schlußfolgerungen über den Zustand eines (beatmeten) Patienten und mögliche gesundheitliche Störungen lassen sich dabei aus der CO₂-Kurvenform ziehen (entsprechende Kurven, bei denen der CO₂-Gehalt (oder CO₂-Partialdruck) gegen die Zeit aufgetragen ist, bezeichnet man auch als Kapnogramme). Bevorzugt wird mit dem dort offenbarten Verfahren der Maximalwert des Kohlendioxidgehalts in der Ausatemluft atemzugaufgelöst bestimmt und angezeigt.

Die vorliegende Erfindung beruht darauf, das in der Patentanmeldung PCT/EP00/03689 beschriebene Verfahren zur Bestimmung des Kohlendioxidgehalts in der Ausatemluft dazu zu verwenden, die Zuverlässigkeit einer Messung zu beurteilen, die mit einem üblichen Atemalkoholmeßgerät durchgeführt wurde.

Bevorzugt ist daher eine Ausführungsform der Erfindung, bei der die CO₂-Kurvenform atemzugaufgelöst aufgezeichnet und am Meßgerät graphisch angezeigt wird. Dies kann erfindungsgemäß auch für mehrere CO₂-Partialdruck-Zeit-Funktionen (Kapnogramme) hintereinander geschehen. Diese Ausführungsform hat den Vorteil, daß geschultes Bedienpersonal an der Kurvenform und auch gegebenenfalls durch Vergleich mehrerer Kurven miteinander erkennen kann, ob die Testperson bewußt oder unbewußt (krankheitsbedingt) verfälschte Ergebnisse bei der Messung der Blutalkoholkonzentration verursacht.

Erfindungsgemäß bevorzugt sind Verfahren und Vorrichtungen zur Bestimmung der Blutalkoholkonzentration, bei denen die Verfahren und Vorrichtungen ähnlich sind, wie in der EP-A 752 584 und der US-A 3,830,630 beschrieben, auf deren Offenbarung insoweit Bezug genommen wird. Allerdings sind diese Verfahren und Vorrichtungen derart abgewandelt, daß statt eines kostspieligen Kohlendioxidsensors ein preiswerter, schneller Sauerstoffsensor verwendet wird und daß der maximale Kohlendioxidgehalt in der Ausatemluft über den mit dem Sauerstoffsensor bestimmten Sauerstoffpartialdruck in der Ausatemluft bestimmt wird.

Erfindungsgemäß wird der Kohlendioxidgehalt damit derart bestimmt, daß der gemessene Sauerstoffpartialdruck in der Ausatemluft von dem Sauerstoffpartialdruck der Umgebungsluft abgezogen wird. Der so ermittelte Wert entspricht dem Kohlendioxidgehalt in der Ausatemluft. Hierbei muß berücksichtigt werden, daß die Ausatemluft in erster Näherung eine Temperatur von 37°C und einen hohen Feuchtigkeitsgehalt aufweist. Insbesondere wird im Rahmen dieser Anmeldung davon ausgegangen, daß die bei der Atemalkoholmessung von der Testperson ausgeatmete Luft eine Temperatur von 37°C und eine relative Feuchtigkeit von 100% aufweist. Diese Temperatur und Feuchtigkeitswerte sind von denen der Umgebungsluft in aller Regel verschieden. Dies sollte bei der Differenzbildung berücksichtigt werden, um die Meßgenauigkeit zu erhöhen.

Der Sauerstoffpartialdruck der Umgebungsluft kann voreingestellt sein, z.B. auf 21 kPa, erfindungsgemäße bevorzugt ist es jedoch, wenn der Sauerstoffpartialdruck der Umgebungsluft unmittelbar vor der Messung des Sauerstoffpartialdrucks in der Ausatemluft bestimmt wird, geeigneterweise mit dem gleichen Sauerstoffsensor, mit dem anschließend der Sauerstoffpartialdruck in der Ausatemluft bestimmt wird.

Erfindungsgemäß ebenfalls bevorzugt ist eine Ausführungsform, bei der ein übliches Atemalkoholmeßgerät, wie es bei Verkehrskontrollen eingesetzt wird, mit einem schnellen Sauerstoffsensor ausgestattet wird. Diese erfindungsgemäß bevorzugte Ausführungsform umfaßt weiterhin noch eine elektronische Schaltung und eine Anzeigevorrichtung, die aus den mit dem schnellen Sauerstoffsensor ermittelten Sauerstoffpartialdrucken in der Ausatemluft den Minimalwert des Sauerstoffpartialdrucks in der Ausatemluft bei jedem Atemzug bestimmt und daraus den Maximalwert des Kohlendioxidgehalts in der Ausatemluft bei jedem Atemzug bestimmt und anzeigt. Hierzu ist es, wie bereits vorstehend ausgeführt, bevorzugt, daß der schnelle Sauerstoffsensor unmittelbar vor der Bestimmung des Sauerstoffpartialdrucks in der Ausatemluft den Sauerstoffpartialdruck in der Umgebungsluft bestimmt, wobei Feuchtigkeits- und Temperaturunterschiede zwischen der Ausatemluft und der Umgebungsluft geeignet zu berücksichtigen sind. Liegt der maximale Wert des Kohlendioxidpartialdrucks unterhalb eines bestimmten Grenzwerts, beispielsweise unterhalb von 4,5%, weiß das Bedienpersonal, daß die Atemalkoholmessung unter Umständen nicht zuverlässig ist. Die Messung kann dann entweder wiederholt werden, oder andere geeignete Maßnahmen können ergriffen werden. Alternativ kann auch, wie es in der EP-A 752 584 oder der US-A 3,830,630 beschrieben ist, eine Atemalkoholmessung nur angezeigt bzw. nur durchgeführt werden, wenn ein bestimmter Grenzwert des Kohlendioxidgehalts in der Ausatemluft überschritten wird.

Das erfindungsgemäße Verfahren, bei dem der Maximalwert des Kohlendioxidgehalts in der Ausatemluft bestimmt wird, hat gegenüber anderen Verfahren, bei denen der Kohlendioxidgehalt zu einem bestimmten Zeitpunkt bestimmt wird oder bei denen nur überprüft wird, ob der Kohlendioxidgehalt in der Ausatemluft einen festgelegten Wert übersteigt, den Vorteil, daß es aussagekräftiger ist und auch dann eine Manipulation einer Atemalkoholmessung feststellt, wenn andere Verfahren versagen. So fällt bei dem erfindungsgemäßen Verfahren auf, wenn die Ausatemluft einen ungewöhnlich hohen Kohlendioxidpartialdruck aufweist, und das Verfahren arbeitet zuverlässig, auch bei solchen Probanden, bei denen das Kapnogramm z.B. krankheitsbedingt eine stark verzerrte Form aufweist.

Die erfindungsgemäßen Vorteile sind natürlich besonders ausgeprägt, wenn bei der Atemalkoholmessung das gesamte Kapnogramm aufgenommen und angezeigt wird, wie dies bei der besonders bevorzugten Ausführungsform der Erfindung der Fall ist.

Erfindungsgemäß vorteilhaft ist es, daß ein schneller Sauerstoffsensor im Prinzip mit bekannten Atemalkoholmeßgeräten zusammenarbeiten kann. Es ist hierfür nur notwendig, noch ein zusätzliches Adapterstück an das Atemalkoholmeßgerät anzuschließen.

Auswerte- und Anzeigevorrichtungen, die die von den Sauerstoffsensoren gelieferten Signale entsprechend verarbeiten und darstellen können, sind im Prinzip bekannt und können von einem Fachmann auf übliche Art und Weise angepaßt und in die erfindungsgemäße Vorrichtung integriert werden.

Sofern das Atemalkoholmeßgerät oder der Sauerstoffsensor mit Temperatur- und/oder Feuchtigkeitssensor gekoppelt ist, kann der hierdurch ermittelte Temperatur- bzw. Feuchtigkeitswert sowohl für die Umgebungsluft als auch für die ausgeatmete Luft selbstverständlich verwendet werden, um den Meßfehler bei der Bestimmung des Sauerstoffpartialdrucks und des daraus errechneten Kohlendioxidpartialdrucks zu verringern. Entsprechende Korrekturformeln sind bekannt.

Für das erfindungsgemäße Verfahren bzw. die erfindungsgemäße Vorrichtung sollte ein schneller Sauerstoffsensor verwendet werden. Bevorzugt sind Sauerstoffsensoren mit einer Ansprechzeit von z.B. 500 Millisekunden oder weniger. Die Verwendung eines schnellen Sauerstoffsensors ist bevorzugt, damit das Minimum des Sauerstoffpartialdrucks bzw. das Maximum des Kohlendioxidgehalts in der Ausatemluft mit guter Genauigkeit bestimmt werden kann.

Werden zusätzlich Kapnogramme aufgezeichnet, so hat ein schneller Sauerstoffsensor den Vorteil, daß die Auflösung der Kapnogramme mit sinkender Ansprechzeit des Sauerstoffsensors steigt.

Schnelle Sauerstoffsensoren, die für das erfindungsgemäße Verfahren geeignet sind, sind bekannt und kommerziell erhältlich. Es können beispielsweise galvanische, paramagnetische oder optische Sauerstoffsensoren verwendet werden. Auch Sauerstoffsensoren, die mit Laserdioden arbeiten, sind bekannt und verwendbar. Aus Kostengründen wird erfindungsgemäß ein schneller elektrochemischer Sauerstoffsensor bevorzugt, wie er beispielsweise von der Firma Teledyne Analytical Instruments und von der Anmelderin vertrieben wird.

Erfindungsgemäß sollte der Sauerstoffsensor möglichst nahe am Mund des zu untersuchenden Patienten angebracht werden, um Meßungenauigkeiten zu vermeiden.

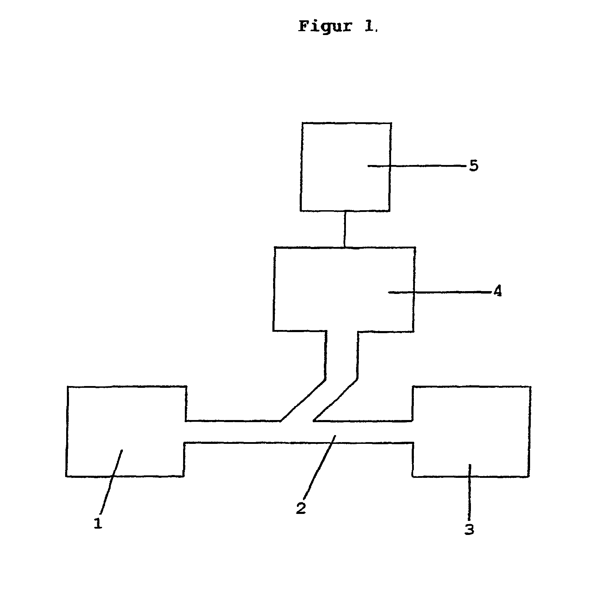

Die Erfindung wird im Folgenden unter Bezugnahme auf Figur 1 näher beschrieben.

Figur 1 zeigt schematisch eine bevorzugte Ausführungsform einer Vorrichtung zur Durchführung des erfindungsgemäßen Verfahrens. In Figur 1 bedeutet Bezugszeichen 1 ein Mundstück, das aus hygienischen Gründen auswechselbar sein sollte und in das die zu untersuchende Person hineinbläst. Bezugszeichen 2 stellt ein Adapterstück dar, das auf ein übliches Atemalkoholmeßgerät aufgesetzt werden kann. Bezugszeichen 3 stellt ein übliches Atemalkoholmeßgerät dar. Bezugszeichen 4 zeigt den Sauerstoffsensor, Bezugszeichen 5 die mit dem Sauerstoffsensor verbundene Anzeige- und Auswertevorrichtung.

Bei der Durchführung des erfindungsgemäßen Verfahrens wird nach Einschalten des Geräts automatisch der Sauerstoffpartialdruck in der Umgebungsluft bestimmt und gespeichert. Anschließend wird die zu untersuchende Person in das Mundstück 1 blasen. Über das Adapterstück 2 wird die von der zu untersuchenden Person kommende Luft einmal an das übliche Atemalkoholmeßgerät 3 geleitet, zum anderen auch an den schnellen Sauerstoffsensor 4. Über die Auswerte- und Anzeigevorrichtung 5 wird der Sauerstoffpartialdruck in der Ausatemluft auf an sich bekannte Art und Weise in den Kohlendioxidgehalt umgerechnet, durch Differenzbildung aus dem Sauerstoffgehalt in der Umgebungsluft und dem Sauerstoffgehalt in der Ausatemluft. Falls das Gerät einen Temperatur- und/oder Feuchtigkeitssensor aufweist, können die exakten Temperatur- bzw. Feuchtigkeitswerte der Umgebungsluft bzw. der Ausatemluft zur Korrektur der gemessenen Sauerstoffpartialdrucke verwendet werden.

Der so ermittelte Kohlendioxidgehalt in der Ausatemluft wird dann angezeigt. Der Maximalwert des Kohlendioxidgehalts in der Ausatemluft bei jedem Atemzug wird ebenfalls ermittelt und angezeigt. Das Bedienpersonal kann dann anhand des Kohlendioxidpartialdrucks in der Ausatemluft entscheiden, ob der mit dem Atemalkoholmeßgerät 3 bestimmte Blutalkoholkonzentrationswert zutreffend ist oder nicht.

In einer alternativen Ausführungsform kann auch eine Verbindung zwischen dem Sauerstoffsensor und dem üblichen Alkoholmeßgerät 3 vorliegen, so daß ein Alkoholwert nur dann angezeigt wird, wenn der Maximalwert des Kohlendioxidgehalts in der Ausatemluft einen bestimmten Wert übersteigt bzw. daß nur in einem solchen Fall die Atemalkoholmessung freigegeben wird.

## Patentansprüche

1. Atemalkoholmeßgerät (3) mit einer Vorrichtung zur Bestimmung des Kohlendioxidpartialdrucks in der Atemluft, **dadurch gekennzeichnet, daß** es sich bei der Vorrichtung zur Bestimmung des Kohlendioxidpartialdrucks in der Atemluft um einen Sauerstoffsensor (4) in Kombination mit einer elektronischen Schaltung handelt, die geeignet ist den Minimalwert des Sauerstoffpartialdrucks in der Ausatemluft zubestimmen und den Absolutwert der Differenz des Minimalwerts des Sauerstoffpartialdrucks in der Ausatemluft und des Sauerstoffpartialdrucks in der Umgebungsluft als Maximalwert des Kohlendioxidpartialdrucks in der Ausatemluft an zuzeigen.

2. Atemalkoholmeßgerät (3) nach Anspruch 1, **dadurch gekennzeichnet, daß** es sich bei dem Sauerstoffsensor (4) um einen elektrochemischen Sauerstoffsensor handelt.

3. Atemalkoholmeßgerät nach Anspruch 1 oder 2, **dadurch gekennzeichnet, daß** die Vorrichtung atemzugaufgelöst die CO₂-Kurvenform bestimmt und anzeigt.

4. Verfahren zur Überprüfung der Zuverlässigkeit einer über ein Atemalkoholmeßgerät bestimmten Blutalkoholkonzentration, bei dem zeitgleich zur Atemalkoholmessung in der Ausatemluft neben der Alkoholkonzentration der Kohlendioxidpartialdruck bestimmt wird, **dadurch gekennzeichnet, daß** der Kohlendioxidpartialdruck dadurch bestimmt wird, daß in der Ausatemluft über einen Sauerstoffsensor der Minimalwert des Sauerstoffpartialdrucks bestimmt wird und der Absolutwert der Differenz des Minimalwerts des Sauerstoffpartialdrucks in der Ausatemluft und des Sauerstoffpartialdrucks in der Umgebungsluft als Maximalwert des Kohlendioxidpartialdrucks in der Ausatemluft angezeigt wird.

5. Verfahren nach Anspruch 4, **dadurch gekennzeichnet, daß** als Sauerstoffsensor ein elektrochemischer Sauerstoffsensor verwendet wird.

6. Verfahren nach Anspruch 4 oder 5, **dadurch gekennzeichnet, daß** atemzugaufgelöst die CO₂-Kurvenform bestimmt und angezeigt wird.

## Claims

1. A breathalyzer (3) with a device for determining the carbon dioxide partial pressure in the respiratory air, **characterized in that** the device for determining the carbon dioxide partial pressure in the respiratory air is an oxygen sensor (4) in combination with an electronic circuit which is suitable for determining the minimum value of the oxygen partial pressure in the exhaled air and for displaying the absolute value of the difference of the minimum value of the oxygen partial pressure in the exhaled air and of the oxygen partial pressure in the surrounding air as the maximum value of the carbon dioxide partial pressure in the exhaled air.

2. The breathalyzer (3) as claimed in claim 1, **characterized in that** the oxygen sensor (4) is an electrochemical oxygen sensor.

3. The breathalyzer as claimed in claim 1 or 2, **characterized in that** the device determines and displays the CO₂ curve shape for each breath.

4. A method for checking the reliability of a blood alcohol concentration which has been determined via a breathalyzer, in which method the carbon dioxide partial pressure is determined at the same time as the alcohol concentration in the exhaled air, **characterized in that** the carbon dioxide partial pressure is determined by the fact that the minimum value of the oxygen partial pressure in the exhaled air is determined using an oxygen sensor, and the absolute value of the difference of the minimum value of the oxygen partial pressure in the exhaled air and of the oxygen partial pressure in the surrounding air is displayed as a maximum value of the carbon dioxide partial pressure in the exhaled air.

5. The method as claimed in claim 4, **characterized in that** the oxygen sensor used is an electrochemical oxygen sensor.

6. The method as claimed in claim 4 or 5, **characterized in that** the CO₂ curve shape is determined and displayed for each breath.

## Revendications

1. Ethylomètre (3) muni d'un dispositif pour la détermination de la pression partielle de dioxyde de carbone dans l'air respiratoire, **caractérisé en ce que** le dispositif pour la détermination de la teneur en dioxyde de carbone dans l'air respiratoire est un analyseur d'oxygène (4) combiné à un circuit électronique, qui convient pour déterminer la valeur minimale de la pression partielle d'oxygène dans l'air expiré et afficher la valeur absolue de la différence de la valeur minimale de la pression partielle d'oxygène dans l'air expiré et de la pression partielle d'oxygène dans l'air ambiant en tant que valeur maximale de la pression partielle de dioxyde de carbone dans l'air expiré.

2. Ethylomètre (3) selon la revendication 1, **caractérisé en ce que** l'analyseur d'oxygène (4) est un analyseur d'oxygène électrochimique.

3. Ethylomètre selon la revendication 1 ou 2, **caractérisé en ce que** le dispositif détermine et affiche la courbe de CO₂ en étalement sur le cycle respiratoire.

4. Procédé de contrôle de la fiabilité d'un taux d'alcoolémie déterminé au moyen d'un éthylomètre, dans lequel, en même temps que la mesure d'alcool dans l'air expiré, on détermine, outre la concentration d'alcool, la pression partielle de dioxyde de carbone, **caractérisé en ce que** la pression partielle de dioxyde de carbone est déterminée **en ce que** l'on détermine dans l'air expiré, au moyen d'un analyseur d'oxygène, la valeur minimale de la pression partielle d'oxygène et que la valeur absolue de la différence de la valeur minimale de la pression partielle d'oxygène dans l'air expiré et de la pression partielle d'oxygène dans l'air ambiant est affichée en tant que valeur maximale de la pression partielle de dioxyde de carbone dans l'air expiré.

5. Procédé selon la revendication 4, **caractérisé en ce que** l'on utilise comme analyseur d'oxygène un analyseur d'oxygène électrochimique.

6. Procédé selon la revendication 4 ou 5, **caractérisé en ce que** l'on détermine et affiche la courbe de CO₂ en étalement sur le cycle respiratoire.
